# EUROPEAN PATENT SPECIFICATION

(11) **EP 0 500 786 B1**
(45) Date of publication and mention of the grant of the patent: **09.08.1995**
(21) Application number: 91900607.2
(22) Date of filing: 09.10.1990
(51) Int. Cl.: C07C 69/96, C07C 68/02

(54) **PROCESS AND CATALYST FOR PRODUCTION OF AROMATIC CARBONATES**
VERFAHREN UND KATALYSATOR ZUR HERSTELLUNG VON AROMATISCHEN CARBONATEN
PROCEDE ET CATALYSEUR DE PRODUCTION DE CARBONATES AROMATIQUES

(30) Priority: 30.10.1989 US 429954
(43) Date of publication of application: 02.09.1992
(73) Proprietor: THE DOW CHEMICAL COMPANY, Midland, Michigan 48640 (US)
(72) Inventor: HARLEY, A., Dale, Midland, MI 48640 (US); KING, Stanley, S., T., Midland, MI 48640 (US); RAND, Cynthia, L., Sanford, MI 48657 (US)
(74) Representative: Sternagel, Hans-Günther, Dr.
(86) International application number: US9005761
(87) International publication number: WO9106526

(56) References cited:
- US-A- 3 234 261
- US-A- 3 251 873

## Description

The present invention relates to a process and a heterogeneous catalyst for the production of aromatic carbonates, and more particularly to a process and a heterogeneous catalyst for the gas phase reaction of aromatic hydroxy compounds with carbonyl halides for the production of aromatic carbonates under elimination of anhydrous hydrogen halide.

Prior art methods for the production of diaryl carbonates have used the interfacial route involving a two phase reaction system, and various homogeneous catalytic systems. The interfacial route involves the neutralization of the aromatic hydroxy compound with caustic and the subsequent reaction of an aqueous solution of the phenate type salt of the aromatic hydroxy compound with a carbonyl halide, usually phosgene. In the case where the desired product is diphenyl carbonate, excess caustic to insure the complete neutralization of phenol results in a loss of about 20 percent of the phosgene. Salt which represents the loss of two chlor/alkali equivalents is produced. As a consequence, the aqueous stream coming from this reaction process requires treatment prior to disposal. Caustic equivalent, include the Group 1, 2, 11 and 12 hydroxides, oxides, carbonates and phosphates.

The prior art alternatives to the above described interfacial route to diaryl carbonates are various homogeneous catalytic processes. As catalysts these processes have used amines and their salts, pentavalent organophosphorous compounds and their salts and organometallic compounds. Anhydrous hydrogen chloride is produced as a side product rather than the waste salt of the interfacial route. This reduces the problem of waste disposal. However, such processes up to now have had the disadvantages of catalyst degradation, and the need to isolate and recycle the catalyst.

U S-A-2,362,865 discloses the use of metal phenates as catalysts in the reaction of phenol and phosgene to form diphenyl carbonate in a process in which the phenol is in the liquid phase. U S-A-3,234,261 relates to the formation of diaryl carbonate from the reaction of various metal oxides with various chloroformates. Related processes are disclosed in French Patent No. 1,361,228 and U S-A-3,234,263, wherein a tertiary amine base is used as a catalyst, and 3,251,873.

U.S.-A-3,251,873 describes a process for preparing a diaryl carbonate by heating anhydrous reaction mixtures comprising a monophenol, a carbonate precursor, and a catalytic amount of at least one metal phenate of said monophenol, said metal being selected from the group consisting of beryllium, magnesium, calcium, strontium, and barium. The reaction mixture is preferably heated under refluxing conditions in an inert organic solvent medium. The phenate catalyst can be formed in situ by simply adding to the reaction system the free metal or inorganic metal salt, whereby the metal might be loaded on a zeolitic molecular sieve.

The use of the process and catalyst of the present invention allows for the economical production of aromatic carbonates, which are used in melt polymerization processes to produce polycarbonate resins. These polycarbonate resins are useful as molding resins in the production of shaped articles by the application of heat or other suitable techniques.

The general objective of the present invention is to avoid the disadvantages of the prior art methods of production of aromatic carbonates. These include the water and salt disposal problem associated with the interfacial method, and catalyst degradation and regeneration problems associated with various homogeneous catalytic systems. This is accomplished by the process of the present invention for the production of aromatic carbonates which comprises contacting an aromatic hydroxy compound with a carbonyl halide in the presence of a catalytic amount of a catalyst which comprises a salt containing at least one metal cation selected from Groups 1, 2, 3, 4, 5, 12, 13, and 14, and a neutralizing number of at least one counter ion, said salt being impregnated on a porous support.

In terms of the new 18 group notation and the first member of a group of the Periodic Table of the Elements, the correspondence of the group numbers in the new notation, the previous IUPAC notation and the first member of the group is as follows:

| | | | | | | | |
|---|---|---|---|---|---|---|---|
| 1 | 2 | 3 | 4 | 5 | 12 | 13 | 14 |
| IA | IIA | IIIA | IVA | VA | IIB | IIIB | IVB |
| H | Be | Sc | Ti | V | Zn | B | C |

For clarity in view of recent changes in the numbering system, the 18 group notation is used herein.

The present invention solves the problem of catalyst isolation and recycling by providing an inert support for the active phase of the catalyst, which, in effect, renders it insoluble. The reaction is conducted under reaction conditions such that the reactive constituents, the aromatic hydroxy compound and the carbonyl halide, remain in the gas phase or are adsorbed on the catalyst. Desorption of the products, the aromatic carbonate and anhydrous hydrogen halide, occurs immediately after reaction in one embodiment of the reaction which employs a fixed bed reactor. In other embodiments which employ a fluidized bed reactor or a circulating fluidized bed reactor desorption of the products occurs at a later stage of the process.

In one aspect, this invention is the process for the production of an aromatic carbonate by reacting an aromatic hydroxy compound with a carbonyl halide in the presence of a catalytic amount of a metal salt wherein metal cation is selected from Groups, 1, 2, 3, 4, 5, 12, 13 and 14, and which contains a neutralizing number of at least one counter ion, and forming the aromatic carbonate by contacting the reactants in the gas phase with a heterogenous catalyst comprising the metal salt being impregnated on a porous support, whereby the reactants remain in the gas phase when not adsorbed on the catalyst.

The process of the present invention can be carried out at temperatures much higher than those achievable for homogeneous catalytic processes: high enough, in fact, that in one embodiment all reactants and products remain in the gas phase when not adsorbed on the catalyst. These high temperatures have a beneficial effect on the kinetics of the chemical reaction involved in the process of this invention.

In another aspect, this invention is the catalyst for the production of an aromatic carbonate comprising a salt containing at least one metal cation selected from Groups 1, 2, 3, 4, 5, 12, 13 and 14, and a neutralizing number of at least one counter ion.

An important part of the present invention is the catalyst used. The catalyst comprises a salt which may contain a wide variety of metal cations and neutralizing counter ions, and a porous support upon which the salt is impregnated so as to render it insoluble. This allows high temperatures to be used in the process of the present invention, high enough so that the reactants, and in some cases the products as well, remain in the gas phase when not adsorbed on the catalyst. This use of a heterogeneous catalyst solves various prior art problems in the use of catalysts to produce diaryl carbonates.

In the salt of the catalyst desirably at least one metal cation is selected from Group 1, 2, 3, 4, 5, 12, 13, or 14. Preferred cations are those from groups 2 and 12, while most preferred are the cations of magnesium, calcium, strontium, barium and zinc. Preferred counter ions include oxide, chloride and mixtures thereof. Oxide may be introduced into a chloride containing catalyst by treating the catalyst at elevated temperatures with methanol or water.

In a more highly preferred embodiment the porous support is impregnated with a salt wherein the anion is an organic anion, such as a carboxylate or a dicarboxylate, for example, oxalate, or a nitrogen containing anion such as nitrate or nitrate. These salts are converted to metal oxides by calcining in air at 550°C.

The support material of the catalyst is desirably a refractory oxide, or other inert material which is porous and stable at high temperatures. Preferred is one or more of silica, alumina, aluminosilicate, carbon, silicon carbide, silicalite, titania or zirconia, and most preferable is silica, alumina or silicon carbide.

The porous support material of the catalyst desirably has a surface area from 50 m² per gram to 500 m² per gram. The average pore radius of the support material is desirably in the range from 5 nm (50 Å) to 30 nm (300 Å), while the particle size of the catalyst is desirably from 25 »m to 1.5 cm. The salt of the catalyst desirably is 1.0 percent to 40 percent by weight of the catalyst, and preferably from 20 percent to 30 percent by weight of the catalyst.

Desirable aromatic hydroxy starting materials are represented by the general formula 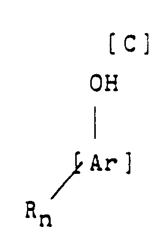
where Ar is an aryl or substituted aryl group with one or more fused rings, R independently selected each occurrence is alkyl, aryl, alkenyl, aryloxy, or alkoxy of 1-12 carbon atoms, and n is an integer. A preferred aromatic hydroxy starting material is represented by the formula 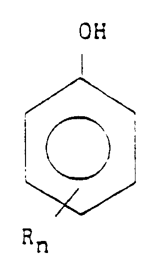
where R independently selected each occurrence is alkyl, aryl, alkenyl, aryloxy, or alkoxy of 1 to 12 carbon atoms, and n is an integer of 0 to 5. More highly preferred are phenols wherein R independently selected each occurrence is alkyl, aryl, alkenyl, aryloxy, or alkoxy of 1 to 6 carbon atoms and n is an integer of 0 to 3. Other desirable aromatic hydroxy starting materials are bisphenols. Highly preferred aromatic hydroxy starting materials are phenol and Bisphenols A and F. A preferred starting carbonyl halide is phosgene.

The process of the present invention may be carried out over a wide range of temperatures from 25°C to 450°C, and at a temperature high enough that the reactants remain in the gas phase when not adsorbed on the catalyst. In a preferable embodiment the reactants and the products of the reaction process remain in the gas phase when not adsorbed on the catalyst. The temperature ranges that are preferred depend, therefore, upon the liquid to vapor transition temperature of the reactants and the products, the pressure at which the process is carried out, and, as an upper limit, the temperature at which degradation of the product occurs. In a preferred embodiment, where the starting materials are phenol and phosgene, and the product is diphenyl carbonate (DPC), the normal boiling point of phenol is 182°C and that of the product diphenyl carbonate is 302°C, so the lower limit of the preferred temperature range for the process at 1.01 x 10² kPa (1 atm) is 182°C and that of the more preferable process is 302°C.

In an alternative embodiment where one or more inert gas is employed in the process the reactants and products remain in the gas phase at temperatures below their boiling points. Desirable inert gases for use in the process of this invention are nitrogen, carbon dioxide, and hydrocarbons, such as toluene. Pressures from 1.01 kPa (0.01 atm) to 5.05 x 10³ kPa (50 atm) may be used, with pressures from 1.01 x 10 kPa (0.1 atm) to 5.05 x 10² kPa (5 atm) being preferred.

A desirable mole ratio of the aromatic hydroxy compound to the carbonyl halide is 2:1, although, as the examples below demonstrate, a wide range of mole ratios may be used. Higher ratios of carbonyl halide relative to the aromatic hydroxy compound result in larger amount of aryl haloformate being formed. From a practical standpoint, it is preferable that the mole ratios be adjusted so that the carbonyl halide, such as highly toxic phosgene, be completely consumed. In that way recycle, removal or further handling of the phosgene or other carbonyl halide is unnecessary. The aromatic hydroxy compound such as phenol is more easily recycled. The aryl haloformate produced may be recycled to produce diaryl carbonate, or it may be removed as desired.

The reaction may be carried out in a fixed bed reactor wherein desirable residence times are from 0.5 sec to 3000 sec with 0.5 sec to 60 sec being preferred, while most preferred is residence time of 0.5 sec to 10 sec. The fixed bed reactor may be operated under reaction conditions such that all reactants and products remain in the gas phase, or in the trickle bed mode such that the diaryl carbonate is recovered as a liquid.

The process of the present invention can be carried out in a fluidized bed reactor or a circulating fluidized bed reactor, in which case the catalyst desirably is utilized as a fluidizable powder.

Periodic regeneration of the catalyst can improve the conversion rate of starting materials to product. Regeneration is accomplished by treating the catalyst with methanol or water at an elevated temperature in the range of 400°C to 600°C.

The following examples are illustrative of the process of the present invention and are in no way intended to limit the scope of the present invention.

### Example 1

20 percent-MgO/SiO₂ catalyst. 54.6 mg, was placed in a quartz reactor. Phenol vapor, ca. 50 mg was passed through the catalyst at 75-100°C over 20 min. 1.0 cm³ of gaseous phosgene was introduced then and brought into contact with the phenol adsorbed on the catalyst. The products of the reaction were collected and separated on a gas chromatograph. The products were identified by comparison to retention times for authentic samples and by mass speotroscopy. The only products detected were phenol and DPC. The quantity of DPC produced was 10 »g.

The catalyst was then heated to 325°C and regenerated with 1.0 cm³ of methanol and subjected to the reaction sequence of phenol and phosgene. This procedure was repeated four times. The average yield of DPC was 1.0 »g DPC/mg MgO.

### Example 2

A 1:2 mixture by weight of MgO and 0.075 mm (200 mesh Tyler Standard) SiC, (230 mg, 78 mg MgO), was placed in a quartz reactor and treated with phenol vapor at 180°C until a steady-state concentration of phenol was observed in the reactor effluent. 3.0 cm³ of gaseous phosgene was introduced then and brought into contact with the phenol adsorbed on the catalyst. The products were analyzed as before and showed that 30 »g of DPC was produced.

Regeneration of the catalyst with methanol was omitted. The catalyst was then sequentially reacted at 180°C with phenol vapor and phosgene four times. The only products detected were phenol and DPC. The average yield of DPC was 0.4 »g DPC/mg MgO.

### Example 3

20.0 cm³ of 20 weight percent MgCl₂/SiO₂ catalyst was placed in a 2.54 cm (1 inch) diameter quartz reactor tube and heated to 200°C. Phenol vapor was introduced at a flowrate of 0.1739 g/min. Phosgene was introduced at a flowrate of 5.83 g/min. The molar ratio of phenol:phosgene was 1:32. N₂ gas was introduced at a flow of 390 cm³/min. The calculated residence time was 2.4 sec. The total amount of phenol introduced was 20.0 g (212.4 meq). The products were condensed into a cold trap and subsequently analyzed by gas chromatography. The results of the analysis, expressed as milliequivalents of phenol, is shown below.

| Compound | Meq Phenol |
|---|---|
| Phenol | 128.39 |
| Phenyl chloroformate | 50.95 |
| Diphenyl carbonate | 20.34 |
| Phenyl salycilate | 0.07 |
| Unknown | 0.16 |
| Total | 199.91 |
| Conv. Phenol | 39.7% |
| Yield Phenyl chloroformate | 60.3% |
| Yield Diphenyl carbonate | 24.1% |
| Mass Bal. Phenol | 93.9% |

### Example 4

Using the same reactor configuration and catalyst amount as described in Example 3, a solution of 0.50 molar phenol and 0.56 molar phosgene in toluene and 380 cm³/min nitrogen carrier gas was introduced. The calculated phenol residence time was 3.2 sec. The analysis of the product is tabulated below.

| | Phenol | Phosgene |
|---|---|---|
| Conversion: | 50.3% | (100 0%) |

| Yield: | | |
|---|---|---|
| Phenyl chloroformate | 16.9% | 8.0% |
| Diphenyl carbonate | 73.8% | 16.6% |
| Material Balance: | 95.4% | 24.2% |

## Claims

1. A process for the production of an aromatic carbonate by reacting an aromatic hydroxy compound with a carbonyl halide in the presence of a catalytic amount of a metal salt wherein the metal cation is selected from Groups 1, 2, 3, 4, 5, 12, 13, and 14, and which contains a neutralizing number of at least one counter ion, and forming the aromatic carbonate by contacting the reactants in the gas phase with a heterogeneous catalyst comprising the metal salt being impregnated on a porous support, whereby the reactants remain in the gas phase when not adsorbed on the catalyst.

2. The process of Claim 1
wherein the metal cation of the catalyst is magnesium ion, calcium ion, strontium ion, barium ion or zinc ion.

3. The process of Claim 1
wherein the counter ion of the catalyst is oxide, chloride, or a mixture thereof, oxalate, nitrate or nitrite.

4. The process of Claim 1
wherein the porous support material for the catalyst is silica, alumina, aluminosilicate, carbon, silicon carbide, silicalite, titania or zirconia.

5. The process of Claim 1
wherein a compound represented by the general formula 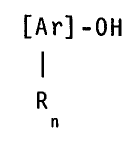 where Ar is an aryl group or a substituted ary group with one or more rings, R independently selected in each occurrence is alkyl, aryl, alkenyl, aryloxy, or alkoxy of 1 to 12 carbon atoms and n is an integer of 0 to 5, is used as aromatic hydroxy compound.

6. The process of any of Claims 1 to 5
wherein phenol is used as aromatic hydroxy compound and phosgene is used as carbonyl halide and the metal cation of the catalyst is selected from one or more of magnesium ion, calcium ion, strontium ion, barium ion, zinc ion and the counter ion is selected from one or more of oxide, chloride, oxalate, nitrate or nitrite.

7. The process of any of Claims 1 to 6
wherein an inert gas selected from nitrogen, carbon dioxide and hydrocarbons is employed.

8. The process of any of Claims 1 to 7
wherein the process is conducted at or above the liquid to vapor transition temperature of the reactants or the aromatic carbonate product.

## Patentansprüche

1. Verfahren zum Herstellen eines aromatischen Carbonats durch Umsetzen einer aromatischen Hydroxyverbindung mit einem Carbonylhalogenid in Gegenwart einer katalytischen Menge eines Metallsalzes, wobei das Kation des Metallsalzes aus Gruppen 1, 2, 3, 4, 5, 12, 13 und 14 ausgewählt ist und das Salz eine neutralisierende Zahl mindestens eines Gegenions enthält, und Ausbilden des aromatischen Carbonats durch Inberührungbringen der Reaktanten in der Gasphase mit einem heterogenen Katalysator, der das Metallsalz in Form eines damit imprägnierten porösen Trägers enthält und bei dem die Reaktanten, wenn sie nicht am Katalysator adsorbiert sind, in der Gasphase verbleiben.

2. Verfahren nach Anspruch 1,
**dadurch gekennzeichnet**,
daß das Metallkation des Katalysators Magnesiumion, Calciumion, Strontiumion, Bariumion oder Zinkion ist.

3. Verfahren nach Anspruch 1,
**dadurch gekennzeichnet**,
daß das Gegenion des Katalysators Oxid, Chlorid oder eine Mischung derselben, Oxalat, Nitrat oder Nitrit ist.

4. Verfahren nach Anspruch 1,
**dadurch gekennzeichnet**,
daß das poröse Trägermaterial für den Katalysator Kieselsäure, Aluminiumoxid, Aluminosilikat, Kohlenstoff, Siliziumcarbid, Silicalit, Titandioxid oder Zirkondioxid ist.

5. Verfahren nach Anspruch 1,
**dadurch gekennzeichnet**,
daß eine Verbindung der allgemeinen Formel 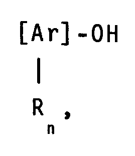 in der Ar eine Arylgruppe oder eine substituierte Arylgruppe mit einem oder mehreren Ringen ist, R unabhängig ausgewählt ist bei jedem Auftreten aus Alkyl, Aryl, Alkenyl, Aryloxy oder Alkoxy mit 1 bis 12 Kohlenstoffatomen und n eine ganze Zahl von 0 bis 5 ist, als aromatische Hydroxyverbindung verwendet wird.

6. Verfahren nach einem der Ansprüche 1 bis 5,
**dadurch gekennzeichnet**,
daß Phenol als aromatische Hydroxyverbindung und Phosgen als Carbonylhalogenid verwendet werden und das Metallkation des Katalysators ausgewählt ist aus einem oder mehreren von Magnesiumion, Calciumion, Strontiumion, Bariumion, Zinkion und das Gegenion ausgewählt ist aus einem oder mehreren von Oxid, Chlorid, Oxalat, Nitrat oder Nitrit.

7. Verfahren nach einem der Ansprüche 1 bis 6,
**dadurch gekennzeichnet**,
daß ein Inertgas, ausgewählt aus Stickstoff, Kohlendioxid und Kohlenwasserstoffen, verwendet wird.

8. Verfahren nach einem der Ansprüche 1 bis 7,
**dadurch gekennzeichnet**,
daß das Verfahren bei oder über der Übergangstemperatur von Flüssigkeit zu Dampf der Reaktanten oder des aromatischen Carbonatproduktes ausgeführt wird.

## Revendications

1. Procédé pour la production d'un carbonate aromatique consistant à faire réagir un composé hydroxyaromatique avec un halogénure de carbonyle en présence d'une quantité catalytique d'un sel métallique dans lequel le cation métallique est choisi dans les groupes 1, 2, 3, 4, 5, 12, 13 et 14, et qui contient une quantité suffisante pour la neutralisation d'au moins un contre-ion, et à former le carbonate aromatique en mettant en contact les réactifs en phase gazeuse avec un catalyseur héterogène comprenant le sel métallique imprégné sur un support poreux, les réactifs restant ainsi en phase gazeuse quand ils ne sont pas adsorbés sur le catalyseur.

2. Procédé selon la revendication 1, dans lequel le cation métallique du catalyseur est l'ion magnésium, l'ion calcium, l'ion strontium, l'ion baryum ou l'ion zinc.

3. Procédé selon la revendication 1, dans lequel le contre-ion du catalyseur est l'oxyde, le chlorure ou leur mélange, l'oxalate, le nitrate ou le nitrite.

4. Procédé selon la revendication 1, dans lequel la matière support poreuse pour le catalyseur est la silice, l'alumine, un aluminosilicate, le carbone, le carbure de silicium, la silicalite, l'oxyde de titane ou la zircone.

5. Procédé selon la revendication 1, dans lequel un composé représenté par la formule générale 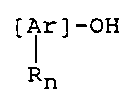 où Ar est un groupe aryle ou un groupe aryle substitué avec un ou plusieurs noyaux, chaque R est, indépendamment, un groupe alkyle, aryle, alcényle, aryloxy ou alcoxy ayant 1 à 12, atomes de carbone et n est un nombre entier de 0 à 5, est utilisé en tant que composé hydroxyaromatique.

6. Procédé selon l'une quelconque des revendications 1 à 5, dans lequel le phénol est utilisé comme composé hydroxyaromatique et le phosgène est utilisé comme halogénure de carbonyle et le cation de métal du catalyseur est choisi parmi un ou plusieurs des ions magnésium, calcium, strontium, baryum, zinc et le contre-ion est choisi parmi un ou plusieurs des contre-ions oxyde, chlorure, oxalate, nitrate ou nitrite.

7. Procédé selon l'une quelconque des revendications 1 à 6, dans lequel on utilise un gaz inerte choisi parmi l'azote, le dioxyde de carbone et les hydrocarbures.

8. Procédé selon l'une quelconque des revendications 1 à 7, dans lequel le procédé est conduit à ou au-dessus de la température de transition de liquide en vapeur des réactifs ou du produit carbonate aromatique.
